# EUROPEAN PATENT APPLICATION

(11) **EP 3 398 595 A1**
(43) Date of publication of application: **07.11.2018**
(21) Application number: 18166347.7
(22) Date of filing: 02.08.2013
(51) Int. Cl.: A61K 31/215, A61K 31/275, A61K 9/00, A61P 29/00, A61K 31/137, A61K 31/47

(54) **COMBINATION THERAPY FOR TREATMENT OF MULTIPLE SCLEROSIS**

(30) Priority: 03.08.2012 EP 12179232; 10.10.2012 EP 12187939; 10.10.2012 US 201261712008 P
(62) Divisional of application: 13745073.0
(71) Applicant: FWP IP APS, 1100 København K (DK)
(72) Inventor: Terwey, Theis, 10119 Berlin (DE); Rupp, Roland, 51467 Bergisch Gladbach (DE); Andersen, Peder M., 1561 Copenhagen V (DK)
(74) Representative: Pohlman, Sandra M.

(57) **Abstract**

The present invention relates to a method of treating MS in a human patient in need of such treatment and comprises administering to said patient a combination therapy in a single oral dosage form (e.g. a tablet or capsule) of dim ethyifurrta rate and one agent selected from teriflunomide (or its prodrug leflunomide), fingolimod and laquinimod. This combination is more effective than the single agents alone and/or has reduced side effects and better tolerability than the single agents alone and/or can be given in a reduced frequency. Moreover, the present invention is directed to a pharmaceutical composition suitable for the oral treatment of multiple sclerosis consisting of dimethylfumarate and one agent selected from teriflunomide, fingolimod and laquinimod as active ingredients and one or more pharmaceutically acceptable excipients.

## Description

### FIELD OF THE INVENTION

The invention relates to pharmaceutical compositions for oral use comprising a fixed combination of a first active pharmaceutical ingredient of dimethylfumarate or a pharmaceutically acceptable administration form thereof and a second active pharmaceutical ingredient selected from teriflunomide, fingolimod and laquinimod or a pharmaceutically acceptable administration form thereof and to the use of such compositions in treating multiple sclerosis. The use of dimethylfumarate in combination with teriflunomide or fingolimod or laquinimod according to this invention allows lowering the dose of dimethylfumarate and/or the agent selected from teriflunomide, fingolimod and laquinimod below levels previously believed to be necessary for efficacy, while achieving better efficacy with comparable adverse effects than seen for the individual agents. Depending on the selected doses the combination therapy can also achieve non-inferior efficacy compared to each of the individual agents when given alone at optimally effective dose but will be associated with less adverse effects compared to the individual agents when given alone at an optimally effective dose. The combinations according to the present invention may also allow for a reduced dosing frequency.

### BACKGROUND

Multiple sclerosis (MS) is a chronic inflammatory disease that attacks myelinated axons in the central nervous system (CNS). It is thought that MS is caused by a T-cell triggered, autoimmune inflammatory reaction with additional B cell activation, involvement of monocytes and macrophages, secretion of cytokines and breakdown of the blood-brain barrier. When myelin is lost nerves can no longer effectively conduct signals which can lead to a plethora of clinical symptoms including sensory defects, motor dysfunctions, visual impairments, bladder and bowel difficulties, sexual dysfunction, fatigue, and even cognitive impairment.

Initially, most cases of MS follow a relapsing-remitting pattern where short episodes of neurologic exacerbations resolve completely but relapses occur (relapsing-remitting MS, RRMS). Later, approximately half of patients develop a continuously progressive pattern with often permanent disability (secondary progressive MS, SPMS). Some cases of MS follow a continuously progressive pattern without remission phases already from the beginning (primary progressive MS, PPMS). Other cases have periods of acute exacerbations while proceeding along a course of increasing neurological deficits without remissions (progressive-relapsing MS, PRMS). The onset of the disease is usually in young adults and it is more common in women. About 2-2.5 million people are living with MS worldwide.

The treatment of choice for exacerbations is generally high doses of corticosteroids. The treatment of the chronic progression of MS aims to target the underlying immune disorder with the goal to reduce the frequency of relapses, to reduce the progression of disability and to preserve brain structure. The available treatments are generally based on immunosuppressive and immunomodulatory mechanisms while for some drugs additional direct neuroprotective effects are postulated.

Treatment success in clinical trials is primarily measured by the reduction in annual relapse rate (ARR) while other commonly used endpoints include time to disability progression as assessed by the Expanded Disability Status Scale (EDSS) or reduction in new brain lesions as measured by brain magnetic resonance imaging (MRI).

All currently available agents are only approved for the relapsing-remitting form of MS. The first agents were all injectable drugs (FDA approved are Interferon beta-la (Avonex, Rebif), Interferon beta-lb (Betaseron, Extavia), Glatiramer acetate (Copaxone) and Natatizumab (Tysabri)) and only recently two oral drugs received an MS label (Fingolimod (Gilenya) in 2010 and teriflunomide (Aubagio) in 2012). In addition, MS is treated with chemotherapeutic agents such as the FDA approved Mitoxantrone (Novantrone) or off-label azathioprine, methotrexate, cladribine and cyclophosphamide.

Besides the approved oral drugs fingolimod and teriflunomide, various other oral agents are in clinical development for MS, the most advanced being dimethylfumarate (Panaclar (BG-12), Biogen Idec), and laquinimod (SAIK-MS, Active Biotech), all having completed Phase III studies.

Dimethylfumarate ("DMF"; trans-1,2-Ethylenedicarboxylic acid dimethyl ester) (Formula 1) belongs to the class of fumaric acid esters (FAE) and appears to have the most attractive safety profile and good efficacy based on two randomized, double-blind, placebo-controlled, dose-comparison Phase III studies with overall more than 2600 patients (DEFINE study (Gold R. et al., N Engl J Med. 2012 Sep 20;367(12):1098-107) and CONFIRM study (Fox RJ et al., N Engl J Med. 2012 Sep 20;367(12):1087-97). Both studies evaluated dimethylfumarate (BG-12) 240 mg twice daily (BID) and three times daily (TID) versus placebo while the CONFIRM study also included an active, reference comparator arm with subcutaneous glatiramer acetate (GA) 20 mg daily.

Regarding efficacy 240 mg BID and 240 mg TID of dimethylfumarate appeared to be superior to the most widely used conventional agents the Interferons (based on indirect comparison) and Glatiramer acetate (based on direct comparison in the CONFIRM trial), but still many patients do experience relapses and progression of disability and may require subsequent therapy with more effective but potentially also more harmful intravenous agents such as Natalizumab (Tysabri, Biogen Idec) or off-label Alemtuzumab (Campath, Sanofi).

Regarding safety, the studies found that the incidence of adverse events, serious adverse events, including serious infections, and discontinuations due to adverse effects were similar across all study groups, including placebo. This excellent safety profile is supported by more than 150.000 patient years experience with another DMF-containing drug, Fumaderm, which has been approved for psoriasis in Germany in 1994 (Morwietz et al., J Dtsch Dermatol Ges, 2007 Aug;5(8):716-7). Despite promising long-term safety data dimethylfumarate is associated with some short term tolerability issues, mainly diarrhea and flushing, which can lead to discontinuation of the drug in some patients.

In more detail, CONFIRM, which analyzed the safety and efficacy of dimethylfumarate 240 mg po (per os = oral) capsule BID or TID vs. placebo vs. glatiramer acetate 20 mg sc (subcutaneously) once-daily in 1430 patients with RRMS, showed that dimethylfumarate met the primary endpoint by significantly reducing annualized relapse rate by 44% and 51% for BID and TID, respectively versus placebo. It also met all secondary relapse and MRI endpoints in both dose regimens. Dimethylfumarate BID and TID reduced the number of new or newly enlarging T2-hyperintense lesions by 71 and 73%, new T1-hypointense lesions by 57 and 65% and the proportion of patients who relapsed by 34 and 45% compared to 54, 41 and 29% for glatiramer acetate, respectively. Dimethylfumarate also reduced 12-week confirmed disability progression as measured by EDSS by 21% for BID and 24% for TID at 2 years compared to 7% for placebo and glatiramer acetate. The most common adverse effects in the dimethylfumarate groups were flushing and gastrointestinal (GI) events. There were no malignancies in the dimethylfumarate groups. The incidence of these events decreased substantially in the dimethylfumarate groups after the 1st month. The most frequently reported serious adverse effect was MS relapse, with no other events reported by more than 2 patients in any group (Press releases, Biogen, and Fox RJ et al., N Engl J Med. 2012 Sep 20;367(12):1087-97).

DEFINE, which analyzed the efficacy and safety of dimethylfumarate 240 po capsules BID and TID in 1237 patients with RRM showed a significant reduction in the proportion of patients with RRMS who relapsed at 2 years compared with placebo (primary endpoint, 49% reduction versus placebo for BID and 50% reduction versus placebo for TID), Both doses of dimethylfumarate showed a significant reduction in annualized relapse rate (53% reduction versus placebo for BID and 48% reduction versus placebo for TID), in the number of new or newly enlarging T2 hyperintense lesions, in new gadolinium-enhancing (Gd+) lesions, and in the rate of disability progression as measured by the Expanded Disability Severity Scale (EDSS) at 2 years (secondary endpoint) (Gold R. et al., N Engl J Med. 2012 Sep 20;367(12):1098-107).

The exact mechanism of action of FAE has not been established but it is generally thought that effects are mediated through depletion in intracellular glutathione (GSH) stores associated with a switch from an inflammatory Th1 to a more anti-inflammatory Th2 immune response, reduction of peripheral CD4+ and CD8+ T-lymphocytes due to apoptosis, and also nuclear factor kappa B (NF-κB)-dependent down-modulation of inflammatory cytokines and adhesion molecule expression (Mrowietz et al, Trends Mol. Med. 2005 Jan;11(1):43-8). More recently, it was proposed that DMF could also act through induction of type II dendritic cells (Ghoreschi et al., J Exp Med. 2011; 208(11):2291-303). Finally, data also suggests a direct anti-oxidant and neuroprotective effect mediated through nrf2 (Gold et al., Clin Immunol. 2012 Jan; 142(1):44-8).

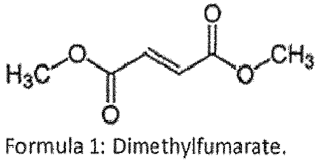

One candidate, teriflunomide (Genzyme) ((Z)-2-cyano-3-hydroxy-but-2-enoic acid-(4'-trifluoromethylphenyl)-amide) (Formula 2) that, in accordance with this invention, may be used in combination with DMF, also has an excellent safety profile in Phase III trials where data on more than 2500 patients has already been presented (TEMSO study, O'Connor et al, N Engl J Med. 201;365(14):1293-303, TENERE study Press release, Sanofi, 20 Dec 2012, TOWER study, Press release, Sanofi, 1 Jun 2012). The most important side effects seen in Phase III trials were diarrhea, hair thinning and elevation of transaminases. Teriflunomide's safety is supported through extensive use of its prodrug leflunomide (Arava) in rheumatoid arthritis since its initial approval in 1998. However, clinical efficacy of teriflunomide against MS was only in the range of the conventional agents (indirect comparison to Interferons and direct comparison Glatiramer acetate) and many patients do experience relapses and progression of disability. In fact, for the 7 mg dose, the TENERE study found a higher relapse rate compared with Interferon and the TOWER study even found no significant difference in 12-week sustained accumulation of disability compared with placebo.
More specifically, TEMSO, a randomized, double-blind, placebo-controlled Phase III trial of teriflunomide 7 mg and 14 mg p.o. once-daily in 1088 RRMS patients showed that teriflunomide 7 mg and 14 mg significantly reduced annualized relapse rate (ARR) by 31.2% and 31.5% at 2 years compared to placebo (primary endpoint). The risk of disability progression was reduced by 24% and 30% for teriflunomide 7 mg and 14 mg, respectively. Teriflunomide also reduced the brain disease activity on a range of magnetic resonance imaging measures including reduction of the burden of disease by 39% and 67% for teriflunomide 7 mg and 14 mg, respectively, compared to placebo. Teriflunomide 7 mg and 14 mg doses were well tolerated, with treatment emergent adverse events including diarrhea, nausea and alanine transferase increases were reported in similar number of patients. No serious opportunistic infections occurred in patients treated with teriflunomide. Further results showed that teriflunomide 7 mg and 14mg significantly increased the time to first relapse by 53.7% and 56.5% during the two years of the study compared to 45.6% on placebo, respectively (TEMSO study, O'Connor et al, N Engl J Med. 201;365(14):1293-303 and Press release, Sanofi-Aventis, 30 Aug 2010 and Press release, Sanofi, 5 Oct 2011).

On the other hand, TENERE, a randomized, open-label Phase III trial in 324 patients with RRMS to assess the effectiveness of 2 doses of teriflunomide 7 mg and 14 mg po tablet once-daily vs interferon-β1a showed no statistical superiority between the Rebif and teriflunomide arms (7 mg and 14 mg) on risk of treatment failure, which was defined as the occurrence of a confirmed relapse or permanent treatment discontinuation for any cause, whichever came first. However, the teriflunomide 7 mg dose showed a higher relapse rate (0.410) than the 14 mg daily dose (0.259) and Rebif (0.216). Most adverse events observed in the teriflunomide arms were mild in severity, including nasopharyngitis, diarrhea, hair thinning, and back pain. These occurred with a higher incidence than in the Rebif arm. The most common adverse events observed in the Rebif arm were increases in alanine aminotransferase levels, headache and flu-like symptoms. These occurred with a higher incidence than in the teriflunomide arms. There were no deaths in the trial (Press release, Sanofi, 20 Dec 2012).

TOWER, a multi-center, randomized, double-blind, placebo-controlled Phase III trial in 1169 RRMS patients, to evaluate 2 doses of teriflunomide 7 mg and 14 mg p.o. tablet once-daily versus placebo showed that patients receiving teriflunomide 14 mg had a significant reduction of 36.3% in annualized relapse rate and 31.5% reduction in the risk of 12wk sustained accumulation of disability compared to placebo. In the 7 mg group a significant reduction in annualized relapse rate was observed compared to placebo but there was no significant difference observed for the risk of 12wk sustained accumulation of disability. The most common types of adverse events reported more frequently in the teriflunomide arms were headache, ALT elevations, hair thinning, diarrhea, nausea and neutropenia (Press release, Sanofi, 1 Jun 2012).
Although in the above mentioned trials the 7 mg dose seemed to have somewhat lower efficacy than the 14 mg dose both doses where approved by the FDA for treatment of RRMS in 2012.

Another Phase III study, TOPIC, is underway in early MS or clinically isolated syndrome. Teriflunomide is also being evaluated together with interferon-B in the Phase III TERACLES trial. With up to 10 years of continuous use in a Phase II extension, teriflunomide has the longest clinical experience of any investigational oral MS therapy.

Teriflunomide was also used in a Phase II combination trial as add-on therapy to IFN where a significant effect on MRI endpoints was observed for 7 mg and 14 mg doses while no significant effect was seen for the reduction in annualized relapse rate (Freedman, Neurology. 2012 Jun 5;78(23):1877-1885). In another Phase II combination trial Teriflunomide at 7 mg or 14 mg added to glatiramer acetate was more effective than placebo added to in reducing Tl-Gd lesions (Freedman et al. Neurology. 2010;74(9):A293.).

Teriflunomide selectively and reversibly inhibits dihydro-orotate dehydrogenase (DHODH), a mitochondrial enzyme required for de novo pyrimidine synthesis. De novo pyrimidine synthesis is required for fast proliferating cells such as activated lymphocytes to meet their needs in DNA, lipid, and sugar metabolism. These effects finally result in strong anti-inflammatory properties through reduced activation and expansion of T- and B-cells in response to autoantigens without apparent cytotoxicity. Teriflunomide has also demonstrated efficiency in inhibiting T-cell-dependent antibody production, suggesting that it modulates the interaction between T cells and B cells. Other effects include reduction of migratory capability of T cells, a diminished ability for exposed T cells to activate monocytes, induction of naïve T cells to favor anti-inflammatory Th-2 differentiation. Cells that rely on DHODH-independent salvage pathways for pyrimidine synthesis (e.g. cells of the hematopoietic system and the gastrointestinal lining) are largely unaffected by teriflunomide's antiproliferative effects.

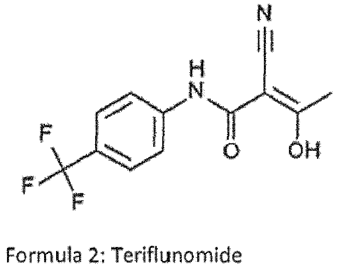

Another drug candidate that may be used in combination with DMF, according to the present invention, is fingolimod (Formula 3). Fingolimod is already approved for RRMS in the US, many European countries and Japan at a dose of 0.5 mg p.o. once daily (Gilenya, Novartis). Fingolimod is an oral sphingosine 1-phosphate receptor (S1PR) modulator which blocks lymphocyte egress from secondary lymphoid organs. After uptake, fingolimod is phosphorylated by sphingosine kinase to the active form which can now bind with high affinity to S1PR. Binding of phosphorylated fingolimod leads to internalization and degradation of the receptor and also to downregulation of S1PR messenger RNA. This results in a decrease of S1PR on the cell surface with consecutive inhibition of lymphocyte egress from lymphoid tissues into the peripheral blood and decreased lymphocyte levels in cerebrospinal fluid (CSF) which finally contributes to reduction of inflammatory events in the central nervous system. In addition to its effects on peripheral blood lymphocytes, it is postulated that fingolimod also has a direct neuroprotective effects through interaction with S1PR on oligodendrocytes, astrocytes, and microglia.

The large Phase 3 clinical trial program of fingolimod in RRMS presented strong efficacy results and an overall acceptable safety profile.

In detail, the 12-months active-comparator Phase III trial TRANSFORMS which randomized 1292 patients with RRMS and a history of at least one relapse to oral fingolimod (0.5 or 1.25mg/day) or intramuscular (i.m.) IFN-b-1a (Avonex, 30µg/week) found that 1-year relapse rates with Fingolimod were 52% (0.5 mg/day) and 38% (1.25 mg/day) lower than with Avonex and that 83% (0. 5 mg/day) and 80% (1.25 mg/day) of patients on fingolimod remained relapse-free vs. only 69% on Avonex. In addition, patients on fingolimod had significantly fewer new or enlarged hyperintense T2 lesions and gadolinium-enhancing T1 lesions on MRI compared with patients on IFN-b-1a. There were no significant differences among groups with respect to EDSS scores. Fingolimod was well tolerated with no significant difference in the overall number of AEs between the fingolimod and the IFN-b-1a group, however the overall number of SAE in the 1.25 mg group seemed to be elevated (10.7% for 1.25 mg vs. 7.0% for 0.5 mg vs. 5.8% for IFN-b-1a). AE leading to the discontinuation of a study medication were also most frequent in the 1.25 mg group (10.0% for 1.25 mg vs. 5.6% for 0.5 mg vs. 3.7% for IFN-b-1a), mainly consisting of bradycardia and atrioventricular block. Overall, there was a transient, dose-dependent reduction in the heart rate that developed within 1 hour after the initial administration of fingolimod, which was consistent with the findings in prior trials. 1% of patients in the 1.25 mg group and 0.5% of patients in the 0.5 mg group developed macular edema. Reflecting fingolimod's mechanism of action lymphocyte counts were reduced after 1 month by 77% in the 1.25 mg group and by 73% in the 0.5 mg group. Mild and moderate upper and lower respiratory tract infections were slightly more frequent among patients receiving fingolimod (Press releases Novartis and Cohen JA et al., N Engl J Med. 2010 Feb 4;362(5):402-15).

The 24-month double-blind, placebo-controlled FREEDOMS trial studied 1272 patients with EDSS scores of 0-5.5, and at least one relapse in the previous year or at least two relapses in the previous 2 years and fingolimod doses of 0.5 mg/day and 1.25 mg/day versus placebo. In this trial Fingolimod reduced the frequency of MS relapses by 54% and 60%, and the risk of disability progression by 30% and 32% confirmed after 3 months during the 24-months period and 37% and 40% confirmed after 6 months during the 24-months period, respectively, vs. placebo. These findings were supported by positive effects on brain lesions on MRI scans. Fingolimod was well tolerated with no difference in the overall number of AE, SAE and deaths between the fingolimod and the placebo group. However, AEs that led to discontinuation of the study drug were more common with fingolimod at a dose of 1.25 mg (14.2% of patients) than with fingolimod at a dose of 0.5 mg (7.5% of patients) or with placebo (7.7% of patients), mainly consisting of bradycardia, atrioventricular conduction block at, macular edema, elevated liver-enzyme levels, and hypertension. Again, as seen in the earlier trial there was an increased risk for episodes of bradycardia in the two fingolimod groups but only after administration of the first dose. Effects on the heart rate and atrioventricular conduction appear to be dose-related and result from the modulation of sphingosine-1-phosphate type 1 receptors in cardiac tissue. Macular edema was diagnosed in seven patients, all of whom were receiving 1.25 mg of fingolimod. Lymphopenia of less than 0.2×10⁹ per liter developed in 5.4%, 3.5% and 0.5% for the 1.25 mg dose, the 0.5 mg dose and placebo, respectively. Lower respiratory tract infections were more common with fingolimod than with placebo, otherwise the incidence of infections was similar (Press releases Novartis and Kappos L et al., N Engl J Med. 2010 Feb 4;362(5):387-401 and Gergely P, Mult Scler 2009;15:Suppl 2:S125-S126.).

The follow-up up study FREEDOMS-II and an extension trial of TRANSFORMS generally confirmed the data discussed above (Press releases Novartis and Kathri B et al., Lancet Neurol. 2011 Jun;10(6):520-9). With an overall lower incidence of adverse events on 0.5 mg and no significant differences in efficacy, this dose was selected as the preferred dosage for further development and was the dose finally approved by regulatory agencies.

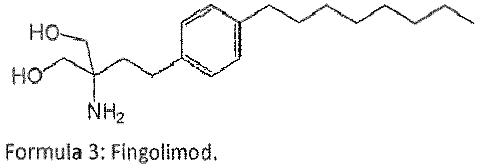

A further candidate which may be combined with DMF, in accordance with the present invention, is laquinimod (Formula 4). Laquinimod has passed two large Phase III studies (ALLEGRO and BRAVO trials with more than 2400 patients with RRMS) and was filed for marketing authorization in the EU in 2012, while a confirmatory Phase III trial (CONCERTO in 1800 RRMS patients) to support the US submission is ongoing (Press releases Teva and Active Biotech and Comi G et al., N Engl J Med. 2012 Mar 15;366(11):1000-9).

In ALLEGRO, the first of the two finished Phase III trials, patients were randomized to receive once-daily oral 0.6 mg laquinimod or matching placebo for 24 months. The primary endpoint was the number of confirmed relapses during the 24-months double blind study period while secondary endpoints included confirmed disability progression and changes in MRI. The study enrolled 1106 RRMS patients (Press releases Teva and Active Biotech and Comi G et al., N Engl J Med. 2012 Mar 15;366(11):1000-9). In ALLEGRO laquinimod showed a statistically significant 23% reduction in ARR (p=0,0024) and a 36% reduction in the risk of EDDS progression (p=0.0122) as well as a 33% significant reduction in progression of brain atrophy (p<0.0001). Laquinimod was generally safe and well-tolerated. The overall frequency of AEs was similar between the active and the placebo groups, with 87% for laquinimod and 81% for placebo. The four most common adverse events in the laquinimod group were ALT (alanine aminotransferase) elevation of greater than 3 times the upper limit of the normal range but less than or equal to 5 times the upper limit (3.6% for laquinimod vs. 0.4% for placebo), abdominal pain (5.8% vs. 2.9%, respectively), back pain (16.4% vs. 9.0%, respectively), and cough (7.5% vs. 4.5%, respectively). SAEs occurred in 11.1% of patients receiving laquinimod and in 9.5% of patients receiving placebo.

The Phase 3 BRAVO trial was again a two-year, randomized, double-blind, placebo-controlled study of a once-daily oral dose of 0.6 mg laquinimod in RRMS patients (n=1331), however this time an exploratory interferon beta-la arm was added. In BRAVO, the primary endpoint of reduction in ARR versus placebo did not reach statistical significance (p = 0.075), and only after running a pre-specified sensitivity analysis to correct for baseline characteristics imbalances between the active and the placebo group, statistical significance was met with a reduced ARR of 21%. In this corrected analysis laquinimod also demonstrated a 34% reduction in the risk of EDSS progression (p=0.044) and a 28% reduction in brain volume loss (p=<0.0001). Safety and tolerability of laquinimod were similar to ALLEGRO with no signal of immunosuppression. With interferon beta-la ARR was reduced by 29% compared to placebo (p = 0.002) and disability progression was reduced by 29% (p = 0.089) while no treatment effect was observed on brain atrophy.

Due to the fact that BRAVO missed its primary endpoint, CONCERTO, a Phase III trial of 0.6 mg or 1.2 mg laquinimod in 1800 RRMS patients was initiated.

Phase II trials had also established clinical efficacy of a lower 0.3 mg dose (Comi G et al., Lancet. 2008 Jun 21;371(9630):2085-92 and Polman C et al., Neurology. 2005 Mar 22;64(6):987-91) however a 0.1 mg dose was not able to significantly affect disease activity as measured by the cumulative number of active CNS lesions (Polman C et al., Neurology. 2005 Mar 22;64(6):987-91).

Regarding mechanism of action, it is thought that laquinimod has immunomodulatory properties within the central nervous system and may also have direct neuroprotective effects (reviewed in Giacomini PS, Clin Immunol. 2012 Jan; 142(1):38-43). Laquinimod's molecular target is not well-defined, however some studies suggest that it can bind S100A9, a calcium binding protein that influences cell signaling. Pre-clinical studies were able to show that laquinimod's effects are in part mediated through decreased Th1 and Th17 responses and an increase in regulatory T cells with reduction of pro-inflammatory cytokines IFN-γ and TNFα while promoting production of the anti-inflammatory cytokines IL-4, IL-10 and TGF-β. In addition laquinimod seems to be able to interfere with lymphocyte migration into the central nervous system through interaction with specific adhesion molecules. Other data suggests that laquinimod may also directly reduce demyelination and induce axonal protection, potentially through upregulation of neurotrophic factors such as brain derived neurotrophic factor (BDNF) (Thöne J, Am J Pathol. 2012 Jan;180(1):267-74 and Schulze-Topphoff U, PLoS One. 2012; 7(3): e33797. Published online on March 30, 2012. doi: 10.1371/journal.pone.0033797).

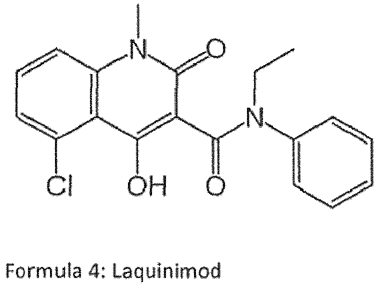

Notwithstanding the above reported works and (partial) progresses, it is still the case that all available agents are only partly effective in halting ongoing inflammatory tissue damage and clinical progression of MS. The reason why therapies are only moderately effective may be seen in the complex and heterogeneous MS pathogenesis where targeting only one aspect of the disease may not suffice to completely stop the disease process. One strategy with the potential to increase treatment efficacy is to combine two or more drugs with distinct modes of action. Such combinations have, for example, been generally described in WO 2007/006307 (salts of fumaric acid monoalkylesters with a multitude of other drugs, see pages 20-25) and specifically claimed in WO 2011/100589 (fumaric acid esters such as dimethylfumarate with either glatiramer acetate or interferon beta). The above described experimental combination therapies with teriflunomide are further examples, but they have not resulted in unequivocally positive results.

Thus, while major advances in MS therapy have already been made, there is still a large unmet need for drugs with improved effectiveness, less side effects, better tolerability and more convenience. The present invention to treat MS with a fixed combination of teriflunomide and dimethylfumarate or combination of fingolimod and dimethylfumarate or combination of laquinimode and dimethylfumarate addresses these needs.

### SUMMARY OF THE INVENTION

This invention is in its broadest aspect is directed toward novel combination of oral agents to treat multiple sclerosis, i.e. a combination of teriflunomide, fingolimod or laquinimod with dimethylfumarate.

Teriflunomide, fingolimod and laquinimod have been selected as powerful partners to be combined with dimethylfumarate due to the partly non-overlapping mechanism of action with dimethylfumarate as well as the generally non-overlapping side-effect profile.

The two active ingredients contained in the combination formulation, i.e. dimethylfumarate in combination with teriflunomide, fingolimod or laquinimod, may be present in any pharmaceutically acceptable administration form of either of them. Such pharmaceutically acceptable administration forms, as used herein, include any pharmaceutically acceptable and therapeutically effective crystalline and non-crystalline forms, solvates or hydrates, and in the case of teriflunomide its Z- and E-enolic forms and mixtures thereof and also its prodrug leflunomide. A further component of the claimed oral pharmaceutical composition according to the present invention is one or more pharmaceutically acceptable excipients. The term "excipient" as used in this application is to be understood broadly and encompasses any pharmaceutically acceptable inactive substance that may be present in an oral pharmaceutical administration form, including (but not limited to) fillers, diluents, binders, matrix formers, disintegrants, lubricants, sustained release agents, coating agents and the like.

This invention also provides a pharmaceutical composition containing dimethylfumarate in combination with teriflunomide or fingolimod or laquinimod as the sole active ingredients, together with one or several pharmaceutically acceptable excipients, which is suitable for once daily administration.

One preferred embodiment of the invention provides for administering a novel fixed-dose combination for once daily oral use of a first active component which is dimethylfumarate, at a dose that is therapeutically effective when used alone, and of a second active component which is teriflunomide, at a dose that has not shown therapeutic efficacy when used alone. Therefore, according to a preferred aspect of the invention, this pharmaceutical composition contains dimethylfumarate at a dose range of 500 mg to 750 mg and teriflunomide at a dose range of 1 mg to 6 mg.

Another preferred embodiment of the invention provides for administering a novel fixed-dose combination for once daily oral use of a first active component which is dimethylfumarate, at a dose that is therapeutically effective when used alone, and of a second active component which is fingolimod, at a dose that has not shown therapeutic efficacy when used alone. Therefore, according to a preferred aspect of the invention, this pharmaceutical composition contains dimethylfumarate at a dose range of 500 mg to 750 mg and fingolimod at a dose range of 0.05 mg to 0.45 mg.

Also, another preferred embodiment of the invention provides for administering a novel fixed-dose combination for once daily oral use of a first active component which is dimethylfumarate, at a dose that is therapeutically effective when used alone, and of a second active component which is laquinimod, at a dose that has not shown therapeutic efficacy when used alone. Therefore, according to a preferred aspect of the invention, this pharmaceutical composition contains dimethylfumarate at a dose range of 500 mg to 750 mg and laquinimod at a dose range of 0.05 mg to 0.25 mg.

According to the present invention, the combination products described herein will show better efficacy (as measured by reduction of annualized relapse rate and/or progression of disability and/or a similarly accepted endpoint) than dimethylfumarate alone. In addition, the inventive combination products will not show an increase in severe adverse events compared with dimethylfumarate and the individual combination partners alone.

A further embodiment of the invention provides for administering a novel fixed-dose combination for once daily oral use of a first component which is dimethylfumarate, in a daily dose below the dally doses shown to be therapeutically effective for MS in DEFINE and CONFIRM studies, and of a second active component which is teriflunomide, fingolimod or laquinimod at a dose that has not shown therapeutic efficacy when used alone. The combination product according to this preferred aspect of the invention will show a non-inferior efficacy (as measured by reduction of annualized relapse rate and/or progression of disability and/or a similarly accepted endpoint) compared with each dimethylfumarate and teriflunomide, fingolimod and laquinimod, respectively, when used at therapeutically effective doses alone but will be associated with less adverse effects compared to the individual agents when given alone at a respective dose. Thus, in a preferred aspect of the present invention the composition contains dimethylfumarate at a dose range of 125 mg to 500 mg and teriflunomide at a dose range of 1 mg to 6 mg. In another preferred aspect of the present invention the composition contains dimethylfumarate at a dose range of 125 mg to 500 mg and fingolimod at a dose range of 0.05 mg to 0.45 mg. In yet another preferred aspect of the present invention the composition contains dimethylfumarate at a dose range of 125 mg to 500 mg and laquinimod at a dose range of 0.05 mg to 0.25 mg.

A further embodiment of the invention provides for administering a novel fixed-dose combination for twice daily oral use of a first active component which is dimethylfumarate, at a dose that is therapeutically effective when used alone, and of a second active component which is teriflunomide, fingolimod or laquinimod, at an absolute daily dose that has not shown therapeutic efficacy when used alone. According to the invention, the combination product will show a significantly better efficacy (as measured by reduction of annualized relapse rate and/or progression of disability and/or a similarly accepted endpoint) then dimethylfumarate alone. In addition, this combination will not show a statistically significant increase in severe adverse events compared with dimethylfumarate alone.

A further embodiment of the invention provides for administering a novel fixed-dose combination for twice daily oral use of a first component which is dimethylfumarate, in a dose below the doses shown to be therapeutically effective in DEFINE and CONFIRM studies, and of a second active component which is teriflunomide, fingolimod or laquinimod at an absolute daily dose that has not shown therapeutic efficacy when used alone. This combination product will show a non-inferior efficacy (as measured by reduction of annualized relapse rate and/or progression of disability and/or a similarly accepted endpoint) compared with each dimethylfumarate and teriflunomide, fingolimod and laquinimod, respectively, when used at therapeutically effective doses alone but will be associated with less adverse effects compared to the individual agents when given alone at a respective dose.

In further embodiments of the invention, teriflunomide is replaced in all of the above combinations by its prodrug leflunomide at a bioequivalent dose (as measured by teriflunomide pharmacokinetics).

### DETAILED DESCRIPTION OF THE INVENTION

This invention is related to a method of treating MS in a human patient in need of such treatment and comprises administering to said patient a combination therapy In a single oral dosage form (e.g. a tablet or capsule) of dimethylfumarate in combination with teriflunomide (or its prodrug leflunomide), fingolimod or laquinimod. The combination formulation is more effective than the single agents alone and/or has reduced side effects and better tolerability than the single agents alone and/or can be given in a reduced frequency.

Although dimethylfumarate as well as teriflunomide, fingolimod and laquinimod have each been used individually for the treatment of MS, the agents have not been used in combination for the treatment of MS. The inventors have recognized that anadded or synergistic effect of dimethylfumarate, on the one hand, and terifunomide, fingolimod or laquinimod, on the other hand, will most likely be due to the fact that dimethylfumarate and the other three agents have different molecular targets and many non-overlapping modes of action in the pathophysiology of MS. Dimethylfumarate acts through depletion of GSH stores and activation of nrf2 which mediates significant neuroprotective properties in addition to its leading immunomodulatory effects with interference with the Th1/Th2 differentiation while no major immunosuppressive effects have been observed. On the other hand Teriflunomide, acting through selective inhibition of dihydro-orotate dehydrogenase (DHODH), fingolimod, acting through downregulation of S1PR on the cell surface, and laquinimod, potentially acting through S100A9, are assumed to have effects on additional aspects of the immune response, such as lymphocyte migration, regulatory T cell responses and antibody production, leading to a potentially more broad inhibitory effect with relevant immunosuppressive activity. According to the present invention, the specific spectrum of activities of these respective agents and the selection of optimal doses allows for a particularly advantageous efficacy and side effect profile of the combination.

In a preferred combination therapy according to the present invention a drug such as teriflunomide, fingolimod or laquinimod at a dose that was not shown to have a significant clinical effect when used alone still has a significant additional effect when used in combination with another drug (dimethylfumarate) at a dose that is effective alone. In addition, the combination will be associated with a similar or even more benign side effect profile compared to the single drugs. Finally, combining a drug usually used in a twice daily regime such as dimethylfumarate with a drug usually used in a once daily regime such as teriflunomide, fingolimod or laquinimod may allow creating a once daily combination drug with non-inferior efficacy to both drugs when used alone and, depending on specific side effect profile and dose, no increase in side effects.

One preferred composition according to the present invention is intended for once daily use and consists of component 1) Dimethylfumarate at a dose range of 500 mg to 750 mg and of component 2) Teriflunomide at a dose range of 1 mg to 6 mg and of components 3) (excipients) which are required for the pharmaceutical formulation. A particularly preferred combination according to this aspect of the invention will contain 625 mg dimethylfumarate and 5 mg teriflunomide, Further preferred combinations contain 500 mg dimethylfumarate and 6 mg teriflunomide, 500 mg dimethylfumarate and 5 mg teriflunomide, 500 mg dimethylfumarate and 4 mg teriflunomide, 500 mg dimethylfumarate and 3 mg teriflunomide, 625 mg dimethylfumarate and 4 mg teriflunomide, 625 mg dimethylfumarate and 3 mg teriflunomide, 625 mg dimethylfumarate and 2 mg teriflunomide, and 625 mg dimethylfumarate and 1 mg teriflunomide. Further preferred embodiments contain 750 mg dimethylfumarate in combination with 1, 2, 3, 4 or 5 mg teriflunomide.

Another preferred composition according to the invention is intended for once daily use and consists of component 1) Dimethylfumarate at a dose range of 125 mg to 500 mg and of component 2) Teriflunomide at a dose range of 1 mg to 6 mg and of components 3) (excipients) which are required for the pharmaceutical formulation. Preferably, the composition according to this aspect of the invention is intended for once daily use and consists of component 1) Dimethylfumarate at a dose range of 125 mg to 375 mg and of component 2) Teriflunomide at a dose range of 1 mg to 6 mg and of components 3) (excipients) which are required for the pharmaceutical formulation. Particularly preferred combinations according to this aspect of the invention contain 375 mg dimethylfumarate in combination with 2, 3, 4, 5 or 6 mg teriflunomide, or 375 mg dimethylfumarate in combination with 1, 2, 3, 4 or 5 mg teriflunomide, or 375 mg dimethylfumarate in combination with 5 mg teriflunomide, or 250 mg dimethylfumarate in combination with 2, 3, 4, 5 or 6 mg teriflunomide, or 125 mg dimethylfumarate In combination with 3, 4, 5 or 6 mg teriflunomide.

A third preferred composition is intended for twice daily use and consists of component 1) Dimethylfumarate at a dose range of 250 mg twice daily to 375 mg twice daily and of component 2) Teriflunomide at a dose range of 0.5 mg twice daily to 3 mg twice daily and of components 3) which are required for the pharmaceutical formulation. A particularly preferred combination according to this aspect of the invention will contain 375 mg dimethylfumarate and 2.5 mg teriflunomide. Further preferred combinations contain 250 mg dimethylfumarate and 3 mg teriflunomide, 250 mg dimethylfumarate and 2.5 mg teriflunomide, 250 mg dimethylfumarate and 2 mg teriflunomide, 250 mg dimethylfumarate and 1.5 mg teriflunomide, 375 mg dimethylfumarate and 2 mg teriflunomide, 375 mg dimethylfumarate and 1.5 mg teriflunomide, 250 mg dimethylfumarate and 1 mg teriflunomide, and 375 mg dimethylfumarate and 0.5 mg teriflunomide. Further preferred embodiments contain 375 mg dimethylfumarate in combination with 0.5, 1, 1.5, 2, or 2.5 mg teriflunomide.

A fourth composition is intended for twice daily use and consists of component 1) Dimethylfumarate at a dose range of 60 mg twice daily to 250 mg twice daily and of component 2) Teriflunomide at a dose range of 0.5 mg twice daily to 3 mg twice daily and of components 3) which are required for the pharmaceutical formulation. A particularly preferred combination according to this aspect of the invention will contain 150 mg dimethylfumarate and 2.5 mg teriflunomide. Further preferred combinations contain 125 mg dimethylfumarate and 5 mg teriflunomide, 150 mg dimethylfumarate and 3 mg teriflunomide, 125 mg dimethylfumarate and 2.5 mg teriflunomide, 125 mg dimethylfumarate and 2 mg teriflunomide, 125 mg dimethylfumarate and 1.5 mg teriflunomide, 150 mg dimethylfumarate and 2 mg teriflunomide, 150 mg dimethylfumarate and 1.5 mg teriflunomide, 125 mg dimethylfumarate and 1 mg teriflunomide, and 150 mg dimethylfumarate and 0.5 mg teriflunomide. Further preferred embodiments contain 180 mg dimethylfumarate in combination with 0.5, 1, 1.5, 2, or 2.5 mg teriflunomide.

Compositions according to the invention that are intended for once or twice daily use according to the present invention include those wherein Teriflunomide is present in the form of bioequivalent doses (as measured by teriflunomide pharmacokinetics) of its prodrug Leflunomide. Teriflunomide forms from leflunomide via rearrangement and ring opening.

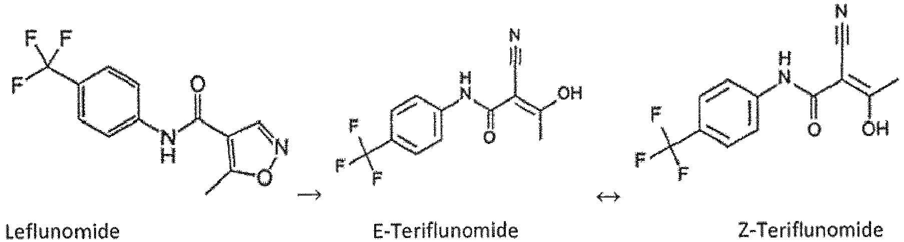

Regarding fingolimod, one preferred composition according to the present invention is intended for once daily use and consists of component 1) Dimethylfumarate at a dose range of 500 mg to 750 mg and of component 2) Fingolimod at a dose range of 0.05 mg to 0.045 mg and of components 3) (excipients) which are required for the pharmaceutical formulation. A particularly preferred combination according to this aspect of the invention will contain 625 mg dimethylfumarate and 0.4 mg fingolimod. Further preferred combinations contain 500 mg dimethylfumarate and 0.4 mg fingolimod, 500 mg dimethylfumarate and 0.3 mg fingolimod, 500 mg dimethylfumarate and 0.2 mg fingolimod, 500 mg dimethylfumarate and 0.1 mg fingolimod, 625 mg dimethylfumarate and 0.3 mg fingolimod, 625 mg dimethylfumarate and 0.2 mg fingolimod, 625 mg dimethylfumarate and 0.1 mg fingolimod, and 625 mg dimethylfumarate and 0.05 mg fingolimod. Further preferred embodiments contain 750 mg dimethylfumarate in combination with 0.05, 0.1, 0.2, 0.3, 0.4, 0.45 mg fingolimod.

Another preferred composition according to the invention is intended for once daily use and consists of component 1) Dimethylfumarate at a dose range of 125 mg to 460 mg and of component 2) Fingolimod at a dose range of 0.05 mg to 0.45 mg and of components 3) (excipients) which are required for the pharmaceutical formulation. Preferably, the composition according to this aspect of the invention is intended for once daily use and consists of component 1) Dimethylfumarate at a dose range of 125 mg to 375 mg and of component 2) Fingolimod at a dose range of 0.05 mg to 0.045 mg and of components 3) (excipients) which are required for the pharmaceutical formulation. Particularly preferred combinations according to this aspect of the invention contain 375 mg dimethylfumarate in combination with 0.05, 0.1, 0.2, 0.3, 0.4, 0.45 mg fingolimod, or 250 mg dimethylfumarate in combination with 0.05, 0.1, 0.2, 0.3, 0.4, 0.45 mg fingolimod, or 125 mg dimethylfumarate in combination with 0.05, 0.1, 0.2, 0.3, 0.4, 0.45 mg fingolimod.

Yet another preferred composition is intended for twice daily use and consists of component 1) Dimethylfumarate at a dose range of 250 mg twice daily to 375 mg twice daily and of component 2) Fingolimod at a dose range of 0.025 mg twice daily to 0.2 mg twice daily and of components 3) which are required for the pharmaceutical formulation. A particularly preferred combination according to this aspect of the invention will contain 375 mg dimethylfumarate and 0.2 mg fingolimod. Further preferred combinations contain 250 mg dimethylfumarate and 0.1 mg fingolimod, 250 mg dimethylfumarate and 0.3 mg fingolimod, 250 mg dimethylfumarate and 0.4 mg fingolimod, 250 mg dimethylfumarate and 0.45 mg fingolimod, 375 mg dimethylfumarate and 0.2 mg fingolimod, 375 mg dimethylfumarate and 0.3 mg fingolimod. Further preferred embodiments contain 375 mg dimethylfumarate in combination with 0.05, 0.1, 0.2, 0.3, 0.4, 0.45 mg fingolimod.

A fourth composition is intended for twice daily use and consists of component 1) Dimethylfumarate at a dose range of 60 mg twice daily to 230 mg twice daily and of component 2) Fingolimod at a dose range of 0.025 mg twice daily to 0.2 mg twice daily and of components 3) which are required for the pharmaceutical formulation. A particularly preferred combination according to this aspect of the invention will contain 150 mg dimethylfumarate and 0.2 mg fingolimod. Further preferred combinations contain 125 mg dimethylfumarate and 0.2 mg fingolimod, 150 mg dimethylfumarate and 0.3 mg fingolimod, 125 mg dimethylfumarate and 0.3 mg fingolimod, 125 mg dimethylfumarate and 0.1 mg fingolimod, 125 mg dimethylfumarate and 0.05 mg fingolimod, 150 mg dimethylfumarate and 0.4 mg fingolimod, 150 mg dimethylfumarate and 0.45 mg fingolimod, 125 mg dimethylfumarate and 0.45 mg fingolimod. Further preferred embodiments contain 180 mg dimethylfumarate in combination with 0.025 mg twice daily to 0.2 fingolimod.

Regarding laquinimod, the following compositions are particularly preferred. One preferred composition according to the present invention is intended for once daily use and consists of component 1) Dimethylfumarate at a dose range of 500 mg to 750 mg and of component 2) Laquinimod at a dose range of 0.05 mg to 0.25 mg and of components 3) (excipients) which are required for the pharmaceutical formulation. A particularly preferred combination according to this aspect of the invention will contain 625 mg dimethylfumarate and 0.25 mg laquinimod. Further preferred combinations contain 500 mg dimethylfumarate and 0.25 mg laquinimod, 500 mg dimethylfumarate and 0.2 mg laquinimod, 500 mg dimethylfumarate and 0.15 mg laquinimod, 500 mg dimethylfumarate and 0.1 mg laquinimod, 625 mg dimethylfumarate and 0.2 mg laquinimod, 625 mg dimethylfumarate and 0.15 mg laquinimod, 625 mg dimethylfumarate and 0.1 mg laquinimod, and 625 mg dimethylfumarate and 0.05 mg laquinimod. Further preferred embodiments contain 750 mg dimethylfumarate in combination with 0.05, 0.1, 0.15, 0.2, and 0.25 mg laquinimod.

Another preferred composition according to the invention is intended for once daily use and consists of component 1) Dimethylfumarate at a dose range of 125 mg to 460 mg and of component 2) Laquinimod at a dose range of 0.05 mg to 0.25 mg and of components 3) (excipients) which are required for the pharmaceutical formulation. Preferably, the composition according to this aspect of the invention is intended for once daily use and consists of component 1) Dimethylfumarate at a dose range of 125 mg to 375 mg and of component 2) Laquinimod at a dose range of 0.05 mg to 0.25 mg and of components 3) (excipients) which are required for the pharmaceutical formulation. Particularly preferred combinations according to this aspect of the invention contain 375 mg dimethylfumarate in combination with 0.05, 0.1, 0.15, 0.2, 0.25 laquinimod, or 250 mg dimethylfumarate in combination with with 0.05, 0.1, 0.15, 0.2, 0.25 mg laquinimod, or 125 mg dimethylfumarate in combination with 0.05, 0.1, 0.15, 0.2, 0.25 mg laquinimod.

Yet another preferred composition is intended for twice daily use and consists of component 1) Dimethylfumarate at a dose range of 250 mg twice daily to 375 mg twice daily and of component 2) Laquinimod at a dose range of 0.025 mg twice daily to 0.125 mg twice daily and of components 3) which are required for the pharmaceutical formulation. A particularly preferred combination according to this aspect of the invention will contain 375 mg dimethylfumarate and 0.125 mg laquinimod. Further preferred combinations contain 250 mg dimethylfumarate and 0.125 mg laquinimod, 250 mg dimethylfumarate and 0.1 mg laquinimod, 250 mg dimethylfumarate and 0.05 mg laquinimod, 250 mg dimethylfumarate and 0.025 mg laquinimod, 375 mg dimethylfumarate and 0.1 mg laquinimod, 375 mg dimethylfumarate and 0.05 mg laquinimod and 375 mg dimethylfumarate in combination with 0.025 mg laquinimod.

Yet another composition is intended for twice daily use and consists of component 1) Dimethylfumarate at a dose range of 60 mg twice daily to 230 mg twice daily and of component 2) Laquinimod at a dose range of 0.025 mg twice daily to 0.125 mg twice daily and of components 3) which are required for the pharmaceutical formulation. A particularly preferred combination according to this aspect of the invention will contain 150 mg dimethylfumarate and 0.125 mg laquinimod. Further preferred combinations contain 125 mg dimethylfumarate and 0.125 mg laquinimod, 125 mg dimethylfumarate and 0.1 mg laquinimod, 125 mg dimethylfumarate and 0.05 mg laquinimod, 125 mg dimethylfumarate and 0.025 mg laquinimod, 150 mg dimethylfumarate and 0.1 mg laquinimod, 150 mg dimethylfumarate and 0.05 mg laquinimod, 150 mg dimethylfumarate and 0.025 mg laquinimod.

According to preferred aspects of the present invention, teriflunomide or fingolimod or laquinimod is used at doses that are below demonstrated therapeutic effectiveness when used alone. Thus, the therapeutic benefit of the preferred inventive combinations is caused by unexpected additional effects provided by teriflunomide or fingolimod or laquinimod at doses heretofore thought to be probably ineffective. Dimethylfumarate has been demonstrated to induce a disease modifying and disease intervening effect as measured on annual relapse rates and progression of disability in patients. Seen in combination with its different (orthogonal) mechanism of action than teriflunomide or fingolimod or laquinimod and in the context of the demonstrated safety, with side effects primarily consisting of mild tolerability issues such as diarrhea, nausea, stomach pain, dimethylfumarate is an ideal partner for combination with teriflunomide or fingolimod or laquinimod. Most pharmaceutical drugs have S-shaped dose-response curves or bell shaped dose response curves. The addition of another pharmacological agent such as teriflunomide or fingolimod or laquinimod would thus be expected to shift the aggregated dose-response curve in a favorable manner and thus be of major clinical and therapeutic utility.

A further aspect of the present invention is a suitable pharmaceutical formulation for once or twice daily oral administration of the inventive combination of dimethylfumarate and teriflunomide or fingolimod or laquinimod. The formulation can be any oral formulation, but is preferably a tablet or pellet formulation, or a capsule formulation, e.g. a gelatin capsule. Tablets, pellets or capsules can be enteric-coated or non-enteric -coated.

According to a particular aspect of this invention, the two active ingredients are present in different portions of the oral formulation that are designed to release the respective active ingredient with different speeds.

Thus, according to this aspect, the invention also provides a pharmaceutical composition for oral use against MS that contains dimethylfumarate and teriflunomide or fingolimod or laquinimod as the active ingredients, wherein the dimethylfumarate is contained in a portion of the composition that provides for prolonged release of the active ingredient and the teriflunomide or fingolimod or laquinimod is contained in a portion of the composition that provides for rapid release of the active ingredient.

In a particular embodiment of the invention the dimethylfumarate is contained in a prolonged release matrix portion of a tablet and the teriflunomide or fingolimod or laquinimod is contained in a coating surrounding the matrix portion. In a particular embodiment, the teriflunomide or fingolimod or laquinimod is contained in an outer enteric coating surrounding the matrix portion of the tablet, which embeds and surrounds the dimethylfumarate. In an alternative embodiment teriflunomide or fingolimod or laquinimod is contained in a separate water-soluble or readily water-disintegratable layer between the core and the outer enteric coating, or as the outermost layer. Suitable tablets according to the invention may contain lactose (e.g. tablettose) or microcrystalline cellulose as a filler, hydroxypropylcellulose or hydroxypropylmethylcellulose as matrix-forming retarding agent and magnesium stearate as a lubricant, and they may be coated, e.g. with a film coat or an enteric coat or a drug-containing layer. Useful coating agents include acrylic polymers, e.g. from the Eudragit series, such as Eudragit L30D and cellulose esters such as hypromellose.

Prolonged or sustained release matrix formulations that are suitable to serve as prolonged release matrix portions of the inventive tablets are disclosed in WO 2010/079222 the disclosure of which is incorporated herein in its entirety. Such prolonged release matrix formulations may be provided with an additional fast release coating containing teriflunomide or fingolimod or laquinimod. Alternatively, teriflunomide or fingolimod or laquinimod may be added to an enteric coating as provided in many of the examples of WO 2010/079222.

Thus, a coated erosion matrix tablet can be used to formulate the combination of dimethylfumarate and teriflunomide or fingolimod or laquinimod according to the present invention. Alternatively, the two active ingredients can also be put into respective controlled release (CR) and immediate release (IR) microtablets or pellets, which can then be filled into gelatin capsules or sachets. In such an embodiment dimethylfumarate will again be in a CR microtablet or pellet, whereas teriflunomide or fingolimod or laquinimod will be in the IR microtablet or pellet.

The DMF core tablets can also consist of 2 layers, one being a controlled release (CR) and a second being an immediate release (IR); the table gives a typical composition for a selected distribution of DMF to CR and IR layer; other distributions of DMF are also possible.

The described 2-layer tablet cores can be coated as the DMF tablets described elsewhere in the patent to yield the combination products.

| *Composition* / *Dose* | *125mg* | *250 mg* | *500 mg* |
|---|---|---|---|
| **CR-Layer** | | | |
| DMF | 60 | 120 | 240 |
| Lactose | 65.85 | 131.70 | 263.40 |
| HPCSL | 6 | 12 | 24 |
| Aerosil | 0.15 | 0.30 | 0.45 |
| Magnesiumstearate | 1 | 2 | 4 |
| *Weight CR* | *133* | *266* | *532* |

| **IR-Layer** | | | |
|---|---|---|---|
| DMF | 65 | 130 | 260 |
| Lactose | 30.85 | 61.7 | 263.40 |
| Avicel | 12 | 24 | 48 |
| HPC SL | 6 | 12 | 24 |
| Crosspovidon | 5 | 10 | 20 |
| Aerosil | 0.15 | 0.30 | 0.45 |
| Magnesiumstearate | 1 | 2 | 4 |
| *Weight CR* | *120* | *240* | *480* |
| | | | |
| *Weight CR* + *IR* | *253* | *506* | *1012* |

### EXAMPLES

The following examples are offered to illustrate various aspects of the invention and are not to be construed as to limit the invention in any way.

### Examples 1-3. Clinical trial design to demonstrate the proposed synergistic effects.

A clinical trial will include multiple sclerosis patients of Remitting-Relapsing type diagnosed on McDonald criteria, with a baseline Expanded Disability Status Scale (EDDS) between 0 and 5 and either at least one relapse within the last 12 months of randomisation and a previous MRI scanning showing lesions consistent with multiple sclerosis or GdE lesions on MRI scan done within 6 months of randomisation. Excluded will be patients with a relapse within 50 days of randomisation or no stabilization from a previous relapse. Patients who within the last year have been treated with T-cell or T-receptor vaccination, total lymphoid irradiation or therapeutic monoclonal antibody treatment, who had been treated with mitoxantron or cyclophosphamide within the last year of randomisation were also excluded. Also patients who within 6 months of randomisation had been treated with cyclosporin, azathioprin, methotrexate or plasmapheresis will beexcluded. Patients with previous gastrointestinal disease such as ulcus duodeni, gastritis or pancreatic disease will be excluded as well. Patients with lymphocytopenia, low white blood ceil count or calculated creatinine clearance of < 60 mL/min at baseline will also be excluded.

The trial will be approved by all relevant Competent Agencies as well as all relevant Ethic Committees.
The trial will be a randomized, double blind, double-dummy, placebo controlled parallel group design testing 3 active treatment arms and a placebo arm:

### Example 1:

1.1: a combination tablet consisting of 500mg prolonged release DMF and the instant release 6 mg teriflunomide in a single formulated enteric coated tablet;
1.2: a teriflunomide 6 mg plus placebo DMF enteric coated tablet;
1.3: a 500mg DMF dose with a teriflunomide placebo enteric coated tablet;
1.4: a placebo DMF and placebo teriflunomide enteric coated tablet.

### Example 2:

2.1: a combination tablet consisting of 500mg prolonged release DMF and the instant release 0.3 mg fingolimod in a single formulated enteric coated tablet;
2.2: a fingolimod 0.3 mg plus placebo DMF enteric coated tablet;
2.3: a 500mg DMF dose with a fingolimod placebo enteric coated tablet;
2.4: a placebo DMF and placebo fingolimod enteric coated tablet.

### Example 3:

3.1: a combination tablet consisting of 500mg prolonged release DMF and the instant release 0.25 mg laquinimod in a single formulated enteric coated tablet;
3.2: a laquinimod 0.25 mg plus placebo DMF enteric coated tablet;
3.3: a 500mg DMF dose with a laquinimod placebo enteric coated tablet;
3.4: a placebo DMF and placebo laquinimod enteric coated tablet.

The placebo arm 1.4, 2.4 and 3.4 will also document the sensitivity of all 3 active arms 1.1-1.3, 2.1-2.3 and 3.1-3.2.

Primary endpoints will be based on MRI scans using the number and volume of new GdE lesions on post contrast T1-weighed sequences as well as the number of T2-weighed enlarged lesions. Secondary endpoints will be the number of relapses monitored monthly and the EDDS that will be assessed at 12 weeks interval from baseline as well as brain atrophy. Safety will be followed closely in particular on differential count of white blood cells, liver enzyme values, gastrointestinal side effects and infections. Laboratory examination will be performed every 4 weeks and general safety as assessed by the reporting of SAE's and AE's and neurological and physical examination. Treatment time will be 24 weeks initially for the evaluation of the primary endpoint followed by a blinded 24 week follow up where the active treatment groups will continue their randomized treatment and the patients on placebo will transferred to active treatment with a continued blinded dosing where they will receive an active combination tablet consisting of 500mg DMF in the prolonged release formulation and the instant release of either teriflunomide, fingolimod or laquinimod in an enteric coated tablet. The number of patients will be 400 with a 1:1:1:1 randomisation between the groups based on previously reported mean MRI lesions reduction data with DMF treatment and treatment with either teriflunomide, fingolimod or laquinimod, assuming a 20% reduction in the number of new GdE lesions, a power of 80% to detect a treatment effect based on a two-sided 5% significance level.

All MRI evaluations will be performed centrally by an experienced neuro-radiologist. An interim analysis is planned after all patients have completed the first 24 weeks comparing each active arm against placebo and furthermore the combination tablet treatment arm compared to each of the single treatment arms. Analysis will be performed using adaptive design and closed analysis with no adjustment of the significance level.

Patients who enter the trial after screening and randomisation will follow a schedule of investigations running at week 2 from randomisation/baseline, at week 4 and then every 4 weeks for the entire trial period with an 8 week follow up for each patient at the end of treatment. Plasma samples for population kinetics will be sampled at baseline, week 4, 8, 12, 24, 36 and 48 of the trial schedule.
Patients who discontinue prematurely will be offered alternative treatment at the discretion of the investigators. An independent Safety Data Monitoring Committee will monitor safety data on a monthly basis, review all SAE's and possible infections and decide on out of schedule laboratory or other safety measures including a for safety reasons premature discontinuation of patients. The trial will be carried out in approximately 50-60 centers in 6-8 countries.

### Examples 4-6. Formulation Examples

An enteric coated tablet containing an erosion matrix core with a film coat is used to formulate the combination of dimethylfumarate and teriflunomide (Example 4), dimethylfumarate and fingolimod (Example 5) and dimethylfumarate and laquinimod (Example 6), respectively, according to the present invention.

The enteric coated tablet consists of an erosion matrix core hosting the dimethylfumarate covered by a film coating hosting teriflunomide (Example 4), fingolimod (Example 5) and laquinimod (Example 6), respectively, and an outer thin enteric coating. The enteric coating rapidly dissolves when reaching the small intestine and releases teriflunomide, fingolimod and laquinimod, respectively, in the duodenum at weakly alkaline pH values. In contrast, due to the erosion matrix the dimethylfumarate is released in a prolonged manner (controlled release) over several hours.

The composition of the tablet core for a 125 mg DMF strength is shown in the following table (same for all Example 4, Example 5, and Example 6).

| Ingredient | Amount of ingredient/275 mg core tablet weight [mg] | Amount of ingredient/core tablet weight [wt.-%] |
|---|---|---|
| Dimethylfumarate | 125 | 45.45 |
| Lactose (Tablettose 100) | 135.7 | 49.35 |
| Hydroxypropylcellulose (HPC-SL) | 12 | 4.36 |
| Silica (Aerosil) | 0.3 | 0.11 |
| Magnesium stearate | 2 | 0.73 |

These 275 mg cores are then coated with 5 wt.-% of an aqueous PVA-solution containing e.g. 5 mg teriflunomide or e.g. 0.3 mg fingolimod or e.g. 0.25 mg laquinimod so as to obtain a teriflunomide-or fingolimod- or laquinimod-containing layer that rapidly dissolves when being contacted with water.

A thin enteric coating is then applied to these coated cores. The coating has the following composition:

| Eudragit L30D55* | 7.56 mg (2.75%**) |
|---|---|
| Triethylcitrate | 0.76 mg |
| Cutina GMS V | 0.23 mg |
| Tween 80 | 0.09 mg |

| | |
|---|---|
| * solid contents are listed (Eudragit is a suspension with 30% solids), ** theoretically applied coating composition, the actually applied coat is approximately 2 wt.-%. | |

Further tablet strengths with varying combinations of a tablet core with prolonged release formulation of DMF containing 60 mg, 125mg, 150 mg, 250 mg, 375 mg and 500 mg combined with 1 mg, 1.5 mg, 2 mg, 2.5 mg, 3 mg, 4 mg, 5 mg and 6 mg of teriflunomide embedded in the film coating will apply. Regarding fingolimod, the following combinations are envisaged: varying combinations of a tablet core with prolonged release formulation of DMF containing 60 mg, 125 mg, 150 mg, 250 mg, 375 mg and 500 mg combined with 0.05 mg, 0.1 mg, 0.2 mg, 0.3 mg, 0.4 mg, 0.45 mg of fingolimod embedded in the film coating. Regarding laquinimod, the following combinations are envisaged: varying combinations of a tablet core with prolonged release formulation of DMF containing 60 mg, 125 mg, 150 mg, 250 mg, 375 mg and 500 mg combined with 0.025 mg, 0.05 mg, 0.1 mg, 0.2 mg, 0.25 mg of laquinimod embedded in the film coating will apply.
The manufacture and coating steps are carried out by known methods such as, e.g., described in WO 2010/079222, examples 21 and 22.

In view of the foregoing, it will be appreciated that the invention described herein inter alia relates to the following items:
1. A pharmaceutical composition suitable for the oral treatment of multiple sclerosis consisting of dimethylfumarate and one agent selected from teriflunomide, fingolimod and laquinimod as active ingredients and one or more pharmaceutically acceptable excipients.
2. Pharmaceutical composition according to item 1, which is suitable for once daily administration.
3. Pharmaceutical composition according to item 2 containing dimethylfumarate at a dose range of 500 mg to 750 mg and teriflunomide at a dose range of 1 mg to 6 mg.
4. Pharmaceutical composition according to item 2 containing dimethylfumarate at a dose range of 500 mg to 750 mg and fingolimod at a dose range of 0.05 mg to 0.45 mg.
5. Pharmaceutical composition according to item 2 containing dimethylfumarate at a dose range of 500 mg to 750 mg and laquinimod at a dose range of 0.05 mg to 0.25 mg.
6. Pharmaceutical composition according to item 3 containing dimethylfumarate at a dose range of 625 mg and teriflunomide at a dose range of 5 mg.
7. Pharmaceutical composition according to item 4 containing dimethylfumarate at a dose range of 625 mg and fingolimod at a dose range of 0.3 mg.
8. Pharmaceutical composition according to item 5 containing dimethylfumarate at a dose range of 625 mg and laquinimod at a dose range of 0.25 mg.
9. Pharmaceutical composition according to item 2 containing dimethylfumarate at a dose range of 125 mg to 500 mg and teriflunomide at a dose range of 1 mg to 6 mg.
10. Pharmaceutical composition according to item 2 containing dimethylfumarate at a dose range of 125 mg to 500 mg and fingolimod at a dose range of 0.05 mg to 0.45 mg.
11. Pharmaceutical composition according to item 2 containing dimethylfumarate at a dose range of 125 mg to 500 mg and laquinimod at a dose range of 0.05 mg to 0.25 mg.
12. Pharmaceutical composition according to item 9 containing dimethylfumarate at a dose range of 375 mg and teriflunomide at a dose range of 5 mg.
13. Pharmaceutical composition according to item 10 containing dimethylfumarate at a dose range of 375 mg and fingolimod at a dose range of 0.3 mg.
14. Pharmaceutical composition according to item 11 containing dimethylfumarate at a dose range of 375 mg and laquinimod at a dose range of 0.25 mg.
15. Pharmaceutical composition according to item 1, which is suitable for twice daily administration.
16. Pharmaceutical composition according to item 15 containing dimethylfumarate at a dose range of 250 mg to 375 mg and teriflunomide at a dose range of 0.5 mg to 3 mg or fingolimod at a dose range of 0.025 mg to 0.20 mg or laquinimod ar a dose range of 0.025 mg to 0.125 mg.
17. Pharmaceutical composition according to item 15 containing dimethylfumarate at a dose range of 375 mg and teriflunomide at a dose range of 2 mg or fingolimod at a dose range of 0.2 mg or laquinimod at a dose range of 0.25 mg.
18. Pharmaceutical composition according to item 15 containing dimethylfumarate at a dose range of 60 mg to 250 mg and teriflunomide at a dose range of 0.5 mg to 3 mg or fingolimod at a dose range of 0.025 mg to 0.20 mg or laquinimod at a dose range of 0.025 mg to 0.125 mg.
19. Pharmaceutical composition according to item 15 containing dimethylfumarate at a dose range of 125 mg and teriflunomide at a dose range of 5 mg or fingolimod at a dose range of 0.2 mg or laquinimod at a dose range of 0.125 mg.
20. Pharmaceutical composition according to any of the preceding items, wherein the dimethylfumarate is contained in a portion of the composition that provides for prolonged release of the active ingredient and one agent selected from teriflunomide, fingolimod and laquinimod is contained in a portion of the composition that provides for rapid release of the active ingredient.
21. Pharmaceutical composition according to item 20, wherein the dimethylfumarate is contained in a prolonged release matrix portion of a tablet and the one agent selected from teriflunomide, fingolimod and laquinimod is contained in a coating surrounding the matrix portion.
22. Pharmaceutical composition according to item 21, wherein the one agent selected from teriflunomide, fingolimod and laquinimod is contained in an outer enteric coating surrounding the matrix portion of the tablet.
23. A method of treating multiple sclerosis (MS) in a human patient in need of such treatment which comprises administering to said patient a pharmaceutical composition for oral use that contains dimethylfumarate and one agent selected from teriflunomide (or its prodrug leflunomide), fingolimod and laquinimod.

## Claims

1. A pharmaceutical composition consisting of dimethylfumarate and one agent selected from teriflunomide, fingolimod and laquinimod as active ingredients and one or more pharmaceutically acceptable excipients.

2. Pharmaceutical composition according to claim 1, which is suitable for once daily administration.

3. Pharmaceutical composition according to claim 1, which is suitable for twice daily administration.

4. Pharmaceutical composition according to any of the preceding claims, wherein the composition is suitable for oral administration, preferably wherein the composition is a tablet, pellet or capsule.

5. Pharmaceutical composition according to any of the preceding claims, wherein the dimethylfumarate is contained in a portion of the composition that provides for prolonged release of the active ingredient and one agent selected from teriflunomide, fingolimod and laquinimod is contained in a portion of the composition that provides for rapid release of the active ingredient.

6. Pharmaceutical composition according to claim 5, wherein the dimethylfumarate is contained in a prolonged release matrix portion of a tablet and the one agent selected from teriflunomide, fingolimod and laquinimod is contained in a coating surrounding the matrix portion.

7. Pharmaceutical composition according to claim 6, wherein the one agent selected from teriflunomide, fingolimod and laquinimod is contained in an outer enteric coating surrounding the matrix portion of the tablet.

8. Pharmaceutical composition according to claim 1, wherein teriflunomide is replaced by its prodrug leflunomide.
